# EUROPEAN PATENT SPECIFICATION

(11) **EP 3 194 006 B1**
(45) Date of publication and mention of the grant of the patent: **01.05.2019**
(21) Application number: 15757367.6
(22) Date of filing: 11.08.2015
(51) Int. Cl.: A61M 25/10

(54) **AUTOMATED INFLATOR FOR BALLOON DILATOR**
AUTOMATISCHE AUFBLASVORRICHTUNG FÜR EINEN BALLONDILATATOR
GONFLEUR AUTOMATISÉ POUR DILATATEUR À BALLONNET

(30) Priority: 29.08.2014 US 201414472448
(43) Date of publication of application: 26.07.2017
(73) Proprietor: Acclarent, Inc., Irvine, CA 92618 (US)
(72) Inventor: LAM, Sivette, Milpitas, CA 95035 (US); LIN, Arthur, M., Fremont CA 94536 (US); CHAMNESS, Scott, O., Menlo Park, CA 94025 (US)
(74) Representative: Carpmaels & Ransford LLP
(86) International application number: PCT/US2015/044653
(87) International publication number: WO 2016/032743

(56) References cited:
- EP-A1- 2 353 632
- EP-A2- 0 581 708
- WO-A2-90/11040
- WO-A2-92/15361
- US-A- 5 273 537
- US-A1- 2011 202 084

## Description

### BACKGROUND

In some instances, it may be desirable to dilate an anatomical passageway in a patient. This may include dilation of ostia of paranasal sinuses (e.g., to treat sinusitis), dilation of the larynx, dilation of the Eustachian tube, dilation of other passageways within the ear, nose, or throat, etc. One method of dilating anatomical passageways includes using a guide wire and catheter to position an inflatable balloon within the anatomical passageway, then inflating the balloon with a fluid (e.g., saline) to dilate the anatomical passageway. For instance, the expandable balloon may be positioned within an ostium at a paranasal sinus and then be inflated, to thereby dilate the ostium by remodeling the bone adjacent to the ostium, without requiring incision of the mucosa or removal of any bone. The dilated ostium may then allow for improved drainage from and ventilation of the affected paranasal sinus. A system that may be used to perform such procedures may be provided in accordance with the teachings of U.S. Pub. No. 2011/0004057, entitled "Systems and Methods for Transnasal Dilation of Passageways in the Ear, Nose or Throat," published January 6, 2011. An example of such a system is the Relieva® Spin Balloon Sinuplasty™ System by Acclarent, Inc. of Menlo Park, California.
European patent publication EP2353632A1 discloses a medical device adapted for local drug delivery comprising a balloon catheter comprising means for a local delivery of a drug functionally connected to at least one controllable automatically driven pump, at least one pressure sensor, and a control unit for controlling the pump depending on a pressure signal of the pressure sensor.

A variable direction view endoscope may be used with such a system to provide visualization within the anatomical passageway (e.g., the ear, nose, throat, paranasal sinuses, etc.) to position the balloon at desired locations. A variable direction view endoscope may enable viewing along a variety of transverse viewing angles without having to flex the shaft of the endoscope within the anatomical passageway. Such an endoscope that may be provided in accordance with the teachings of U.S. Pub. No. 2010/0030031, entitled "Swing Prism Endoscope," published February 4, 2010. An example of such an endoscope is the Acclarent Cyclops™ Multi-Angle Endoscope by Acclarent, Inc. of Menlo Park, California.

While a variable direction view endoscope may be used to provide visualization within the anatomical passageway, it may also be desirable to provide additional visual confirmation of the proper positioning of the balloon before inflating the balloon. This may be done using an illuminating guidewire. Such a guidewire may be positioned within the target area and then illuminated, with light projecting from the distal end of the guidewire. This light may illuminate the adjacent tissue (e.g., hypodermis, subdermis, etc.) and thus be visible to the naked eye from outside the patient through transcutaneous illumination. For instance, when the distal end is positioned in the maxillary sinus, the light may be visible through the patient's cheek. Using such external visualization to confirm the position of the guidewire, the balloon may then be advanced distally along the guidewire into position at the dilation site. Such an illuminating guidewire may be provided in accordance with the teachings of U.S. Pub. No. 2012/0078118, entitled "Sinus Illumination Lightwire Device," published March 29, 2012. An example of such an illuminating guidewire is the Relieva Luma Sentry™ Sinus Illumination System by Acclarent, Inc. of Menlo Park, California.

It may be desirable to provide easily controlled inflation/deflation of a balloon in dilation procedures, including procedures that will be performed only by a single operator. While several systems and methods have been made and used to inflate an inflatable member such as a dilation balloon, it is believed that no one prior to the inventors has made or used the invention described in the appended claims.

### BRIEF DESCRIPTION OF THE DRAWINGS

While the specification concludes with claims which particularly point out and distinctly claim the invention, it is believed the present invention will be better understood from the following description of certain examples taken in conjunction with the accompanying drawings, in which like reference numerals identify the same elements and in which:
FIG. 1 depicts a side elevational view of an exemplary dilation catheter system;
FIG. 2 depicts a side elevational view of an exemplary illuminating guidewire suitable for use with the dilation catheter system of FIG. 1;
FIG. 3 depicts a side cross-sectional view of the illuminating guidewire of FIG. 2;
FIG. 4 depicts a perspective view of an exemplary endoscope suitable for use with the dilation catheter system of FIG. 1;
FIG. 5 depicts a side elevational view of the distal end of the endoscope of FIG. 5, showing an exemplary range of viewing angles;
FIG. 6 depicts a diagrammatic view of an exemplary inflator system suited for use with the dilation catheter system of FIG. 1;
FIG. 7 depicts a front view of an exemplary inflator system suited for use with the dilation catheter system of FIG. 1;
FIG. 8 depicts a diagrammatic view of an exemplary graphical user interface of the system of FIG. 7;
FIG. 9A depicts a front view of certain components of a fluid pressure regulator assembly of the system of FIG. 7;
FIG. 9B depicts a front view of the fluid pressure regulator assembly of FIG. 9A;
FIG. 10A depicts a cross-sectional view, along line 10-10 of FIG. 9A, of certain components of the fluid pressure regulator assembly of FIG. 9A in a deactivated state;
FIG. 10B depicts a cross-sectional view, along line 10-10 of FIG. 9A, of the certain components of the fluid pressure regulator assembly of FIG. 9A in an activated state;
FIG. 11 depicts a flowchart showing steps carried out during an exemplary method of using the inflator system of FIG. 7;
FIG. 12 depicts a flowchart showing steps carried out during another exemplary method of using the inflator system of FIG. 7;
FIG. 13 depicts a flowchart showing steps carried out during another exemplary method of using the inflator system of FIG. 7;
FIG. 14 depicts a flowchart showing additional steps carried out during an exemplary method of using the inflator system of FIG. 7;
FIG. 15 depicts a flowchart showing other additional steps carried out during an exemplary method of using the inflator system of FIG. 7; and
FIG. 16 depicts a flowchart showing other additional steps carried out during an exemplary method of using the inflator system of FIG. 7.

The drawings are not intended to be limiting in any way, and it is contemplated that various embodiments of the invention may be carried out in a variety of other ways, including those not necessarily depicted in the drawings. The accompanying drawings incorporated in and forming a part of the specification illustrate several aspects of the present invention, and together with the description serve to explain the principles of the invention; it being understood, however, that this invention is not limited to the precise arrangements shown.

### DETAILED DESCRIPTION

The following description of certain examples of the invention should not be used to limit the scope of the present invention. Other examples, features, aspects, embodiments, and advantages of the invention will become apparent to those skilled in the art from the following description, which is by way of illustration, one of the best modes contemplated for carrying out the invention. As will be realized, the invention is capable of other different and obvious aspects, all without departing from the invention. Accordingly, the drawings and descriptions should be regarded as illustrative in nature and not restrictive.

It will be appreciated that the terms "proximal" and "distal" are used herein with reference to a clinician gripping a handpiece assembly. Thus, an end effector is distal with respect to the more proximal handpiece assembly. It will be further appreciated that, for convenience and clarity, spatial terms such as "top" and "bottom" also are used herein with respect to the clinician gripping the handpiece assembly. However, surgical instruments are used in many orientations and positions, and these terms are not intended to be limiting and absolute.

It is further understood that any one or more of the teachings, expressions, versions, examples, etc. described herein may be combined with any one or more of the other teachings, expressions, versions, examples, etc. that are described herein. The following-described teachings, expressions, versions, examples, etc. should therefore not be viewed in isolation relative to each other. Various suitable ways in which the teachings herein may be combined will be readily apparent to those of ordinary skill in the art in view of the teachings herein. Such modifications and variations are intended to be included within the scope of the claims.

### I. Overview of Exemplary Dilation Catheter System

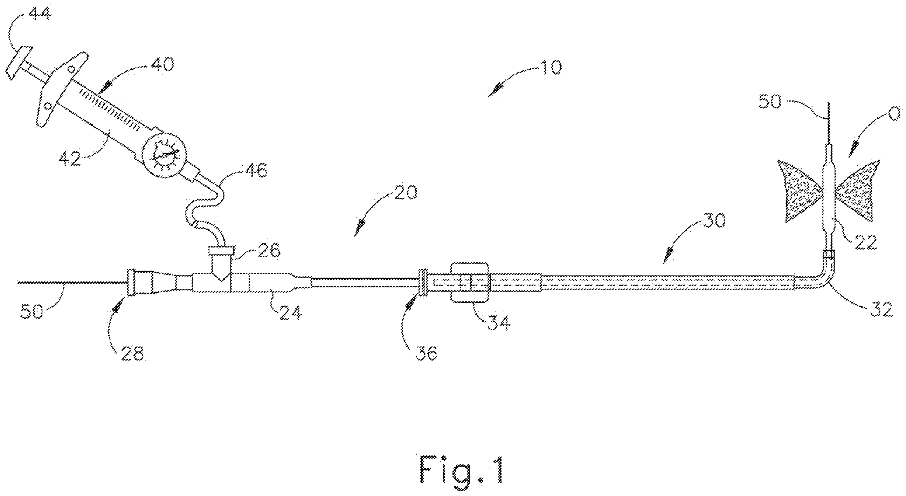

FIG. 1 shows an exemplary dilation catheter system (10) that may be used to dilate the ostium of a paranasal sinus; or to dilate some other anatomical passageway (e.g., within the ear, nose, or throat, etc.). Dilation catheter system (10) of this example comprises a dilation catheter (20), a guide catheter (30), an inflator (40), and a guidewire (50). By way of example only, dilation catheter system (10) may be configured in accordance with at least some of the teachings of U.S. Patent Pub. No. 2011/0004057. In some versions, at least part of dilation catheter system (10) is configured similar to the Relieva® Spin Balloon Sinuplasty™ System by Acclarent, Inc. of Menlo Park, California.

The distal end of dilation catheter (20) includes an inflatable dilator (22). The proximal end of dilation catheter (20) includes a grip (24), which has a lateral port (26) and an open proximal end (28). Dilation catheter (20) includes a first lumen (not shown) that provides fluid communication between lateral port (26) and the interior of dilator (22). Dilation catheter (20) also includes a second lumen (not shown) that extends from open proximal end (28) to an open distal end that is distal to dilator (22). This second lumen is configured to slidably receive guidewire (50). The first and second lumens of dilation catheter (20) are fluidly isolated from each other. Thus, dilator (22) may be selectively inflated and deflated by communicating fluid along the first lumen via lateral port (26) while guidewire (50) is positioned within the second lumen. In some versions, dilation catheter (20) is configured similar to the Relieva Ultirra™ Sinus Balloon Catheter by Acclarent, Inc. of Menlo Park, California. In some other versions, dilation catheter (20) is configured similar to the Relieva Solo Pro™ Sinus Balloon Catheter by Acclarent, Inc. of Menlo Park, California. Other suitable forms that dilation catheter (20) may take will be apparent to those of ordinary skill in the art in view of the teachings herein.

Guide catheter (30) of the present example includes a bent distal end (32) and a grip (34) at its proximal end. Grip (34) has an open proximal end (36). Guide catheter (30) defines a lumen that is configured to slidably receive catheter (20), such that guide catheter (30) may guide dilator (22) out through bent distal end (32). In some versions, guide catheter (30) is configured similar to the Relieva Flex™ Sinus Guide Catheter by Acclarent, Inc. of Menlo Park, California. Other suitable forms that guide catheter (30) may take will be apparent to those of ordinary skill in the art in view of the teachings herein.

Inflator (40) of the present example comprises a barrel (42) that is configured to hold fluid and a plunger (44) that is configured to reciprocate relative to barrel (42) to selectively discharge fluid from (or draw fluid into) barrel (42). Barrel (42) is fluidly coupled with lateral port (26) via a flexible tube (46). Thus, inflator (40) is operable to add fluid to dilator (22) or withdraw fluid from dilator (22) by translating plunger (44) relative to barrel (42). In the present example, the fluid communicated by inflator (40) comprises saline, though it should be understood that any other suitable fluid may be used. There are various ways in which inflator (40) may be filled with fluid (e.g., saline, etc.). By way of example only, before flexible tube (46) is coupled with lateral port (26), the distal end of flexible tube (46) may be placed in a reservoir (218) containing the fluid. Plunger (44) may then be retracted from a distal position to a proximal position to draw the fluid into barrel (42). Inflator (40) may then be held in an upright position, with the distal end of barrel (42) pointing upwardly, and plunger (44) may then be advanced to an intermediate or slightly distal position to purge any air from barrel (42). The distal end of flexible tube (46) may then be coupled with lateral port (26).

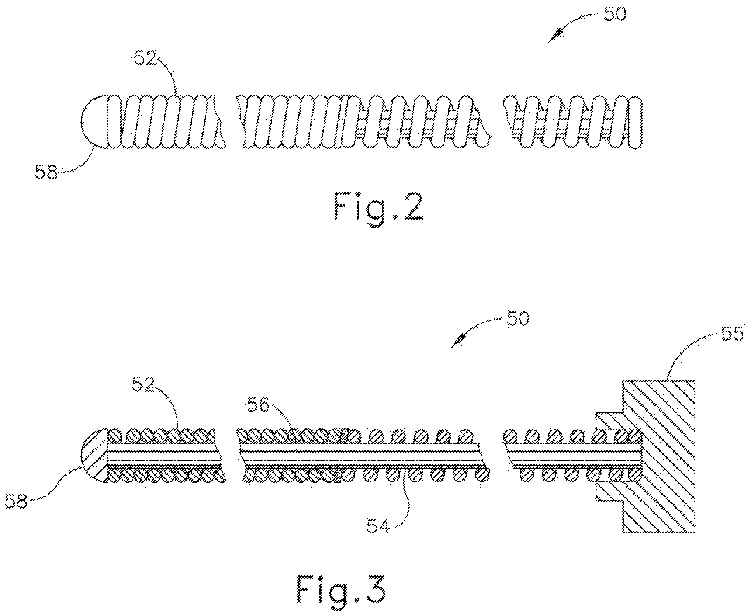

As best seen in FIGS. 2-3, guidewire (50) of the present example comprises a coil (52) positioned about a core wire (54). An illumination fiber (56) extends along the interior of core wire (54) and terminates in an atraumatic lens (58). A connector (55) at the proximal end of guidewire (50) enables optical coupling between illumination fiber (56) and a light source (not shown). Illumination fiber (56) may comprise one or more optical fibers. Lens (58) is configured to project light when illumination fiber (56) is illuminated by the light source, such that illumination fiber (56) transmits light from the light source to the lens (58). In some versions, the distal end of guidewire (50) is more flexible than the proximal end of guidewire (50). Guidewire (50) has a length enabling the distal end of guidewire (50) to be positioned distal to dilator (22) while the proximal end of guidewire (50) is positioned proximal to grip (24). Guidewire (50) may include indicia along at least part of its length (e.g., the proximal portion) to provide the operator with visual feedback indicating the depth of insertion of guidewire (50) relative to dilation catheter (20). By way of example only, guidewire (50) may be configured in accordance with at least some of the teachings of U.S. Pub. No. 2012/0078118. In some versions, guidewire (50) is configured similar to the Relieva Luma Sentry™ Sinus Illumination System by Acclarent, Inc. of Menlo Park, California. Other suitable forms that guidewire (50) may take will be apparent to those of ordinary skill in the art in view of the teachings herein.

In an exemplary dilation procedure, guide catheter (30) may first be positioned near the targeted anatomical passageway, such as a sinus ostium (O). Dilator (22) and the distal end of guidewire (50) may be positioned within or proximal to bent distal end (32) of guide catheter (30) at this stage. Guide catheter (30) is initially inserted into the nose of the patient and is advanced to a position that is within or near the ostium (O) to be dilated. This positioning of guide catheter (30) may be performed under visualization provided by an endoscope such as endoscope (60) described below. After guide catheter (30) has been positioned, the operator may advance guidewire (50) distally through guide catheter (30) such that a distal portion of the guidewire (50) passes through the sinus ostium (O) and into the sinus cavity. The operator may illuminate illumination fiber (56) and lens (58), which may provide transcutaneous illumination through the patient's face to enable the operator to visually confirm positioning of the distal end of guidewire (50) with relative ease.

With guide catheter (30) and guidewire (50) suitably positioned, dilation catheter (20) is advanced along guidewire (50) and through bent distal end (32) of guide catheter (30), with dilator (22) in a non-dilated state until dilator (22) is positioned within the sinus ostium (O) (or some other targeted anatomical passageway). After dilator (22) has been positioned within the ostium (O), dilator (22) may be inflated, thereby dilating the ostium. To inflate dilator (22), plunger (44) may be actuated to push saline from barrel (42) of inflator (40) through dilation catheter (20) into dilator (22). The transfer of fluid expands dilator (22) to an expanded state to open or dilate the ostium (O), such as by remodeling the bone, etc., forming ostium (O). By way of example only, dilator (22) may be inflated to a volume sized to achieve about 10 to about 12 atmospheres. Dilator (22) may be held at this volume for a few seconds to sufficiently open the ostium (O) (or other targeted anatomical passageway). Dilator (22) may then be returned to a non-expanded state by reversing plunger (44) of inflator (40) to bring the saline back to inflator (40). Dilator (22) may be repeatedly inflated and deflated in different ostia and/or other targeted anatomical passageways. Thereafter, dilation catheter (20), guidewire (50), and guide catheter (30) may be removed from the patient.

In some instances, it may be desirable to irrigate the sinus and paranasal cavity after dilation catheter (20) has been used to dilate an ostium (O). Such irrigation may be performed to flush out blood, etc. that may be present after the dilation procedure. By way of example only, such irrigation may be carried out in accordance with at least some of the teachings of U.S. Pub. No. 2008/0138128, entitled "Methods, Devices and Systems for Treatment and/or Diagnosis of Disorders of the Ear, Nose and Throat," published July 31, 2008. An example of an irrigation catheter that may be fed through guide catheter (30) to reach the irrigation site after removal of dilation catheter (20) is the Relieva Vortex® Sinus Irrigation Catheter by Acclarent, Inc. of Menlo Park, California. Another example of an irrigation catheter that may be fed through guide catheter (30) to reach the irrigation site after removal of dilation catheter (20) is the Relieva Ultirra® Sinus Irrigation Catheter by Acclarent, Inc. of Menlo Park, California. Of course, irrigation may be provided in the absence of a dilation procedure; and a dilation procedure may be completed without also including irrigation.

### II. Overview of Exemplary Endoscope

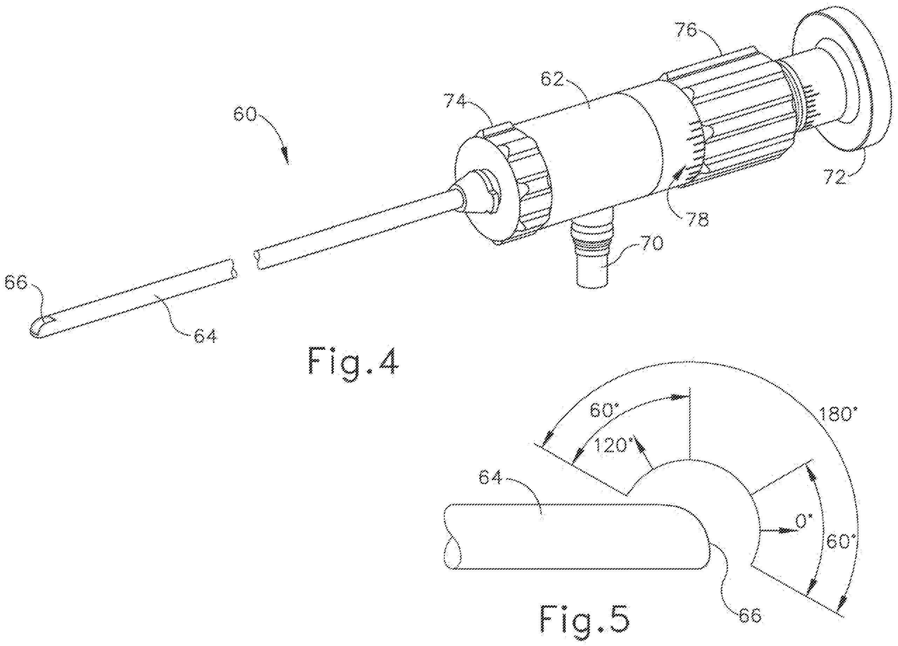

As noted above, an endoscope (60) may be used to provide visualization within an anatomical passageway (e.g., within the nasal cavity, etc.) during a process of using dilation catheter system (10). As shown in FIGS. 4-5, endoscope of the present example comprises a body (62) and a rigid shaft (64) extending distally from body (62). The distal end of shaft (64) includes a curved transparent window (66). A plurality of rod lenses and light transmitting fibers may extend along the length of shaft (64). A lens is positioned at the distal end of the rod lenses and a swing prism is positioned between the lens and window (66). The swing prism is pivotable about an axis that is transverse to the longitudinal axis of shaft (64). The swing prism defines a line of sight that pivots with the swing prism. The line of sight defines a viewing angle relative to the longitudinal axis of shaft (64). This line of sight may pivot from approximately 0 degrees to approximately 120 degrees, from approximately 10 degrees to approximately 90 degrees, or within any other suitable range. The swing prism and window (66) also provide a field of view spanning approximately 60 degrees (with the line of sight centered in the field of view). Thus, the field of view enables a viewing range spanning approximately 180 degrees, approximately 140 degrees, or any other range, based on the pivot range of the swing prism. Of course, all of these values are mere examples.

Body (62) of the present example includes a light post (70), an eyepiece (72), a rotation dial (74), and a pivot dial (76). Light post (70) is in communication with the light transmitting fibers in shaft (64) and is configured to couple with a source of light, to thereby illuminate the site in the patient distal to window (66). Eyepiece (72) is configured to provide visualization of the view captured through window (66) via the optics of endoscope (60). It should be understood that a visualization system (e.g., camera and display screen, etc.) may be coupled with eyepiece (72) to provide visualization of the view captured through window (66) via the optics of endoscope (60). Rotation dial (74) is configured to rotate shaft (64) relative to body (62) about the longitudinal axis of shaft (64). It should be understood that such rotation may be carried out even while the swing prism is pivoted such that the line of sight is non-parallel with the longitudinal axis of shaft (64). Pivot dial (76) is coupled with the swing prism and is thereby operable to pivot the swing prism about the transverse pivot axis. Indicia (78) on body (62) provide visual feedback indicating the viewing angle. Various suitable components and arrangements that may be used to couple rotation dial (74) with the swing prism will be apparent to those of ordinary skill in the art in view of the teachings herein. By way of example only, endoscope (60) may be configured in accordance with at least some of the teachings of U.S. Pub. No. 2010/0030031. In some versions, endoscope (60) is configured similar
to the Acclarent Cyclops™ Multi-Angle Endoscope by Acclarent, Inc. of Menlo Park, California. Other suitable forms that endoscope (60) may take will be apparent to those of ordinary skill in the art in view of the teachings herein

### III. Exemplary Alternative Inflators

Inflator (40) shown in FIG. 1 and described above is just one example of an inflator that may be incorporated into dilation catheter system (10). Additional merely illustrative examples of alternative forms that inflator (40) may take will be described in greater detail below. It should be understood that these exemplary alternative inflators may be readily coupled with flexible tube (46) in place of inflator (40) described above, for use in dilation catheter system (10). In some versions, the exemplary alternative inflators described below may be directly coupled with lateral port (26), such that flexible tube (46) is simply omitted. Other suitable configurations and arrangements will be apparent to those of ordinary skill in the art in view of the teachings herein.

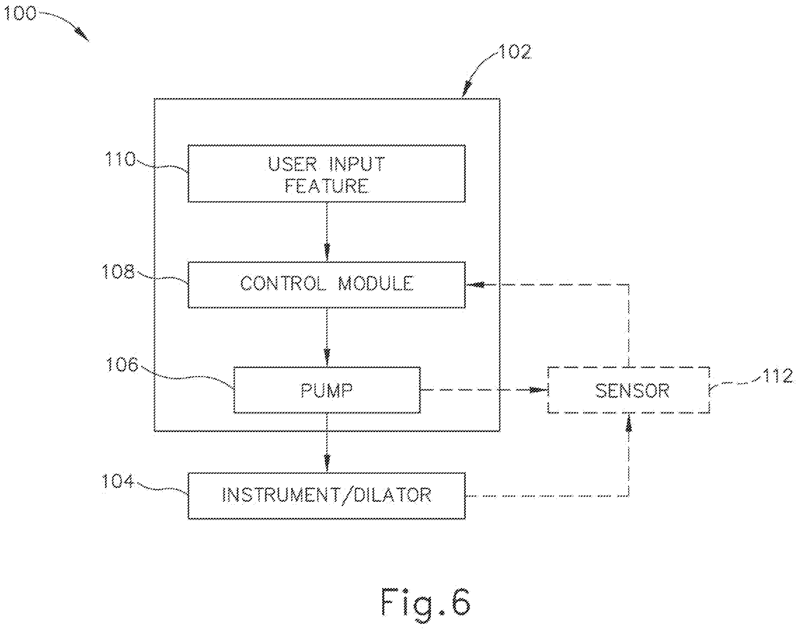

FIG. 6 shows a diagrammatic view of an exemplary dilator system (100) including an inflator assembly (102) and dilation catheter (104). Dilation catheter (104) may or may not be configured in accordance with the teachings of the dilation catheter (20) described herein. Inflator assembly (102) of this example is operable to regulate the pressure of fluid within dilation catheter (104). Inflator assembly (102) includes pump (106), a control module (108) operable to selectively drive the pump (106), and a user input feature (110) operable to selectively activate the control module (108). While these components (106, 108, 110) are shown within the same box representing inflator assembly (102), it should be understood that these components may be physically separate or located and/or integrated in various ways. Dilator system (100) also includes an optional sensor (112), which may comprise a pressure sensor, a force sensor, and/or another type of sensor. Sensor (112) may be part of inflator assembly (102), or may be a separate part of the dilator system (100). In the example shown, sensor (112) may be in fluid communication with one of, or both of, the pump (106) and dilation catheter (104). As shown, dilation catheter (104) is coupled in fluid communication with pump (106) to receive fluid from pump (106).

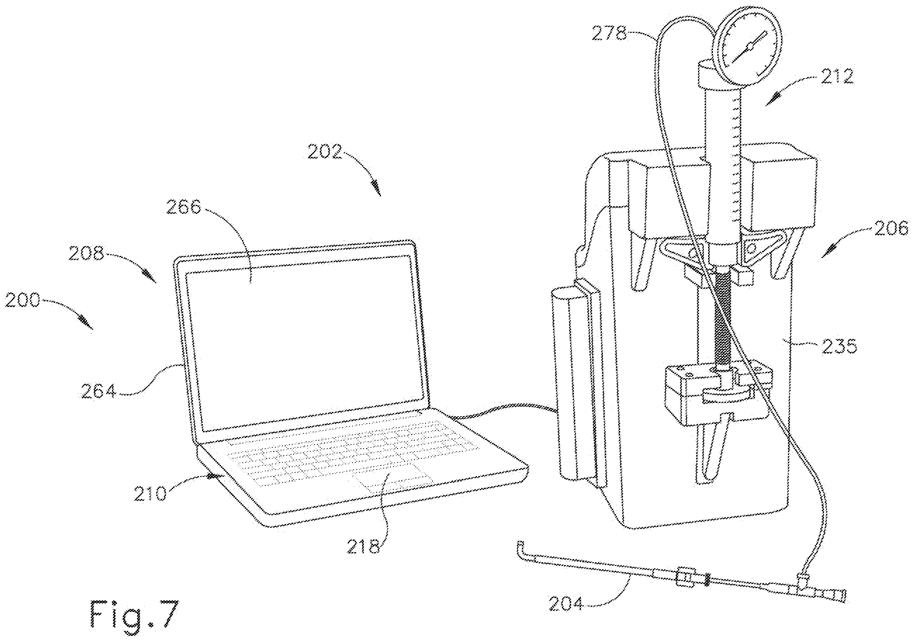

FIGS. 7, 9A-B, and 10A-B show an exemplary dilator system (200) that includes an inflator assembly (202) and a dilation catheter (204). Dilation catheter (204) may or may not be configured in accordance with the teachings of the dilation catheter (20) described herein. Therefore, where appropriate, same or similar components of dilation catheter (204) are indicated with the same reference numerals used with respect to dilation catheter (20), without further explanation. Inflator assembly (202) includes a pump (206), a control module (208) operable to selectively drive the pump (206), and a user input feature (210) operable to selectively activate the control module (208). As described herein, inflator assembly (202) is one merely illustrative example of a form that inflator assembly (102) may take.

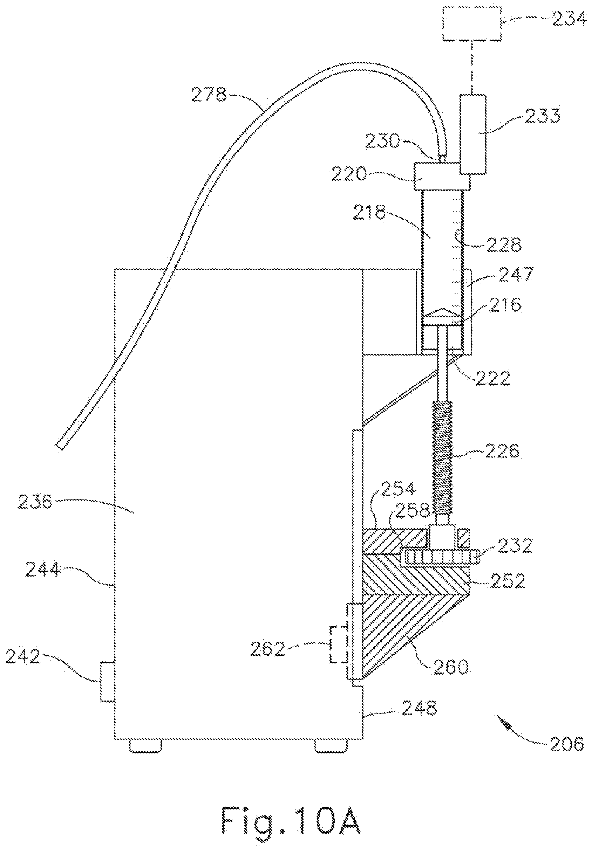

As shown, in this example, pump (206) comprises a syringe assembly (212), which includes a syringe barrel (214) and a syringe plunger (216) that is configured to move within the barrel (214). Barrel (214) comprises a reservoir (218), a distal port (220) (FIGS. 10A-B), a proximal opening (222), and proximal grips (224). A rod (226) extends into barrel (214) and is coupled to plunger (216). Particularly, plunger (216) is coupled to a distal end of rod (226) and extends radially outwardly to the inner wall (228) of barrel (214) to form a substantially fluid tight seal with an inner wall (228) of barrel (214). The volume between plunger (216) and the distal end (230) of barrel (214) forms reservoir (218). By way of example only, reservoir (218) may be configured to hold about 5cc to about 100cc of fluid (e.g., saline). Alternatively, reservoir (218) may have any other suitable capacity. Rod (226) and plunger (216) may translate proximally and distally to adjust the size of reservoir (218). When rod (226) and plunger (216) translate proximally, the volume of reservoir (218) increases. When rod (226) and plunger (216) translate distally, the volume of reservoir (218) decreases. Port (220) at the distal end (230) of barrel (214) is in fluid communication with reservoir (218) such that fluid may flow into and out of reservoir (218) via port (220). Port (220) may be coupled with flexible tube (278) of dilation catheter (204) system. Rod (226) includes a distal handle feature (232) having generally circular shape in cross section. In an alternative example, plunger (216), rod (226), and handle feature (232) may be an essentially integral structure.

Pump (206) of the present example further comprises a gauge (233) positioned distal of reservoir (218) to measure the pressure of fluid within dilator system (200). Gauge (233) may include a pivoting pin that indicates fluid pressure based on the angular position of the pin. Alternatively, gauge (233) may provide any other suitable type of indication of fluid pressure, including but not limited to the other types of fluid pressure indication described below. In the present example, gauge (233) is operable to indicate pressure levels up to at least about 12 atmospheres. For instance, some uses of dilator system (200) may include inflation of dilator (22) to a range between about 4 atmospheres and about 18 atmospheres (or more particularly to a range between about 10 atmospheres and about 12 atmospheres) in order to sufficiently dilate a targeted anatomical passageway. Gauge (233) may thus provide the operator with real time feedback indicating the fluid pressure within dilator (22) to enable the operator to determine whether the desired pressure level has been achieved. An optional pressure sensor (234) may be mounted adjacent to gauge (233) or elsewhere within system (200) and may be in communication with control module (208). Pressure sensor (234) is configured to sense the pressure of fluid in the closed hydraulic circuit that is formed by syringe assembly (212), tubing (278), and dilation catheter (204).

Pump (206) further comprises a drive mechanism (235) that includes features for supporting certain portions of the syringe assembly (212) and driving or causing the movement of certain other features of the syringe assembly (212). In that regard, drive mechanism (235) includes a main body (236), a first engagement feature (238), and a second engagement feature (240). Main body (236) includes one or more inlets and/or ports (242) on a rear portion (244) thereof that allow the drive mechanism (235) to be in wired communication with, for example, user input feature (210) and control module (208), for example, and also with power sources, sensors, etc. First engagement feature (238) includes a pair of flanges (246) that extend outwardly from a front wall (248) of main body (236) and a pair of grounding features (247). Rod (226) extends along a space (250) defined between the flanges (246). Each grip (224) is fixedly coupled between a respective one of the flanges (246) and a respective one of the grounding features (247), thereby fixedly coupling barrel (214) to main body (236) to prevent movement of barrel (214) as plunger (216) is advanced and retracted.

In some versions, each grip (224) may be snap fit or friction fitted between a respective flange (246) and grounding feature (247). In some alternative examples, flanges (246) and/or grounding features (247) may be vertically movable in order to selectively clamp the grips (224) between flanges (246) and grounding features (247). In some such versions, the vertical movement of flanges (246) and/or grounding features (247) is manually actuated. For instance, flanges (246) and/or grounding features (247) may be manually movable along a ratcheting assembly that releasably maintains a selective relative positioning of flanges (246) and grounding features (247). In some versions where flanges (246) and/or grounding features (247) are driven vertically by a motor, solenoid, or other power source, this movement may be manually initiated and/or stopped by an operator depressing a button or otherwise interacting with user input feature (210). In some other versions, a sensor may be configured to sense the presence of grips (224) between flanges (246) and grounding features (247), which may trigger a motor, solenoid, or other power source to automatically move flanges (246) and/or grounding features (247) to clamp onto grips (224). Other suitable ways in which grips (224) may selectively engage flanges (246) and grounding features (247) will be apparent to those of ordinary skill in the art in view of the teachings herein.

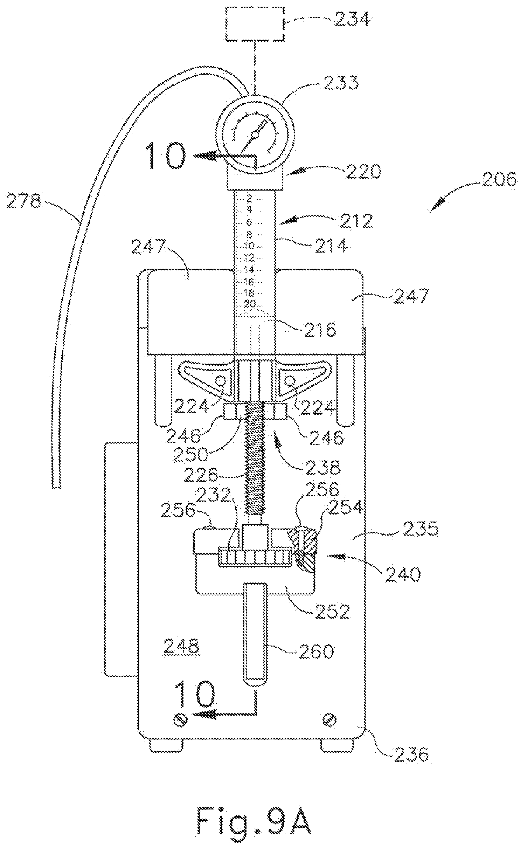

Second engagement feature (240) comprises a first, lower portion (252), and a second, upper portion (254) that are coupled via a plurality of fasteners (256). When coupled together, the first and second portions (252, 254) define a space (258) for the insertion of at least a portion of handle feature (232). As shown, first and second portions (252, 254) partially envelop handle feature (232). In an alternative example, first and second portions (252, 254) may envelop handle feature (232) to a lesser degree, or may substantially completely envelop handle feature (232). In the example shown, first portion (252) includes, or is coupled to, a downwardly extending flange (260). Second engagement feature (240) is coupled to an actuator feature (262) (shown schematically in FIGS. 10A-B) via flange (260). Actuator feature (262) is operable to move the second engagement feature (240) in a first, upward direction and a second, downward direction (as viewed in FIGS. 9A-B). Thus, actuator (262) is operably coupled to and configured to drive the plunger (216) of syringe assembly (212) in response to inputs from a control module (208).

In the example shown, actuator (262) is a linear actuator, the linear motion of which results in linear motion of the second engagement feature (240) relative to main body (236) and first engagement feature (238). However, in alternative examples, the actuator (262) may be a rotary or other type of actuator, configured such that rotation of actuator (262) is converted into linear motion of the first engagement feature (238). By way of example, actuator (262) may comprise one or more solenoids, a motor driven pinion and rack, a motor driven worm or screw gear, a motor driven cable and pulley, a motor driven sprocket and chain, a hydraulic assembly driven by liquid (e.g., water, oil, etc.), a pneumatic assembly driven by a gas (e.g., air, carbon dioxide, etc.), and/or any other kinds of drive features. Various other forms that actuator (262) may take will be apparent to those of ordinary skill in the art in view of the teachings herein.

As shown in FIG. 7, control module (208) of the present example comprises a laptop computer (264) having a storage feature (not shown) such as hard drive that is configured to store one or more fluid pressure profiles, described in further detail below. Control module (208) is operable to selectively drive the pump (206) in accordance with a selected fluid pressure profile. For instance, control module (208) may include one or more processors (e.g., microprocessors, etc.) that are operable to execute the one or more fluid pressure profiles stored in the storage feature. Control module (208) may communicate inputs to pump (206), according to the selected fluid pressure profile, after receiving inputs from an operator via user input feature (210). As shown in FIG. 7, user input feature (210) is also provided through laptop computer (264), such that control module (208) and user input feature (210) are both provided through laptop computer (264) in this example. User input feature (210) includes a monitor screen (266), a mouse pad (268), and a keyboard (270).

As shown, computer (264) is in wired communication with drive mechanism (235) of pump (206) via a plurality of wires. However, in alternative examples, computer (264) or some other kind of other control module (208) may be in wireless (e.g., Bluetooth) communication with drive mechanism (235).

The storage feature of computer (264) is configured to store one or more fluid pressure profiles. The fluid pressure profiles may provide various kinds of control algorithms that are executed by control module (208) to perform a dilation procedure in a patient. The one or more fluid pressure profiles may provide delivery of fluid to dilation catheter (204) until a particular level of pressure of fluid is reached within the dilation catheter (204). For example, the fluid pressure profiles may be associated with a range of cross-sectional dimensions (e.g., diameter) or other features of the expanded dilator (22) that are achieved according to certain pressure levels being met within the dilator (22) or other portions of the dilator system (200). The fluid pressure profiles may, in addition or in the alternative, be associated with certain instrument or instrument(s), such as various kinds of dilation catheters. In that regard, because different instruments such as dilation catheters may include different dimensions, qualities (e.g. compliance of dilator material), etc., the fluid pressure profiles may vary based on the particular kind of instrument being used. Fluid pressure profiles could also vary based on the clinical setting in which dilator system (200) is being used (e.g. the particular anatomical passageway being dilated, etc.), patient specific parameters (e.g. measured ostium size, etc.), and/or other factors.

In some versions, inflator assembly (202) is configured for use in at least the following three clinical contexts: dilation of ostia and/or other drainage passageways for paranasal sinuses, dilation of Eustachian tubes, and dilation of a patient's airway (e.g., larynx or trachea, etc.). Inflator assembly (202) may thus provide separate fluid pressure profiles (or separate groupings of fluid pressure profiles) that are based on a clinical context selected by the operator. By way of further example only, one or more particular fluid pressure profiles may provide a relatively slow rate of inflation to reduce trauma. As another merely illustrative example, one or more particular fluid pressure profiles may provide a relatively slow rate of deflation to improve clinical results (e.g., in versions where dilator (22) is used to dilate a Eustachian tube, etc.).

Inflator assembly (202) may also provide a high speed deflation mode, which may be used to rapidly draw fluid from dilator (22) (e.g., in an emergency scenario when deflator (22) needs to be removed from the patient's airway or other passageway, etc.). System (200) may enable the operator to activate such a high speed deflation mode while system (200) is otherwise in the middle of inflating/deflating dilator (22) while performing in a normal pressure profile operational mode. In other words, system (200) may enable the operator to "bail out" of a normal pressure profile operation, in response to the operator actuating a dedicated input to activate the high speed deflation operation. A rapid deflation of dilator (22) may enable the operator to withdraw dilator (22) from the patient rapidly with a reduced risk of a still-inflated dilator (22) becoming lodged within an anatomical passageway of the patient.

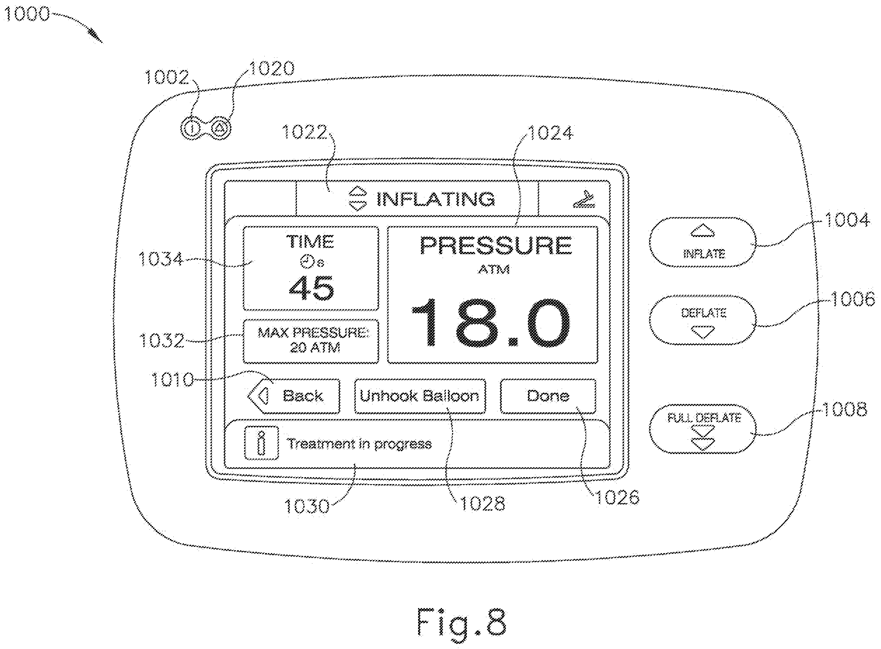

User input feature (210) provides a variety of options that an operator may select from in order to operate the dilator system (200), in accordance with stored fluid pressure profiles and/or other control algorithms. FIG. 8 shows one merely illustrative example of a graphical user interface (GUI) (1000) that may be provided as user input feature (210) (or as a portion of user input feature (210)). In some versions, GUI (1000) is presented through computer monitor (266) of laptop computer (264). Of course, GUI (1000) may alternatively be provided through any other suitable kind of hardware. By way of further example only, GUI (1000) may be presented through a dedicated console that is custom made for dilator system (200). In some such versions, the console may include buttons, a touch screen, and/or various other kinds of user input features.

As shown in FIG. 8, GUI (1000) of the present example includes several input features (e.g., buttons, etc.) that may be activated by an operator; as well as several feedback indicators that are configured to provide information to the operator. In particular, GUI (1000) includes a power button (1002), an inflation button (1004), a deflation button (1006), a rapid deflation button (1008), a back button (1010), a warning indicator (1020), an inflation status indicator (1022), a pressure indicator (1024), a process completion indicator (1026), an unhook balloon indicator (1028), a treatment in progress indicator (1030), a maximum pressure indicator (1032), and an inflation duration indicator (1034). Each of these features will be described in greater detail below. It should be understood, however, that these are all merely illustrative examples. GUI (1000) may include various other features in addition to or in lieu of those shown in FIG. 8. The features shown in FIG. 8 may be modified, substituted, supplemented, or omitted as desired.

Power button (1002) is operable to selectively turn inflator assembly (202) on and off.

Inflation button (1004) is operable to activate pump (206) to drive fluid toward dilation catheter (204) to thereby inflate dilator (22). By way of example only, inflation button (1004) may cause control module (208) to automatically execute a selected fluid pressure profile in response to a single actuation of inflation button (1004). In addition or in the alternative, inflation button (1004) may enable the operator to manually control the inflation of dilator (22). For instance, inflation button (1004) may activate pump (206) to drive fluid toward dilation catheter (204) to thereby inflate dilator (22) for however long the operator holds inflation button (1004) in a depressed state. In some such versions, once the operator releases inflation button (1004), inflator assembly (202) may stop inflating dilator (22) and maintain the fluid pressure in dilation catheter (204) until the operator provides further input. It should also be understood that inflation button (1004) may provide different responses based on how the operator activates inflation button (204). For instance, inflation button (1004) may cause control module (208) automatically execute a selected fluid pressure profile in response to the operator quickly tapping inflation button (1004) one or two times; and provide manual inflation of dilator (22) in response to the operator holding inflation button (204) in a depressed state. Other suitable ways in which inflation button (1004) may operate will be apparent to those of ordinary skill in the art in view of the teachings herein.

Deflation button (1006) is operable to activate pump (206) to draw fluid from dilation catheter (204) to thereby deflate dilator (22). By way of example only, deflation button (1006) may cause control module (208) to automatically execute a selected fluid pressure profile in response to a single actuation of deflation button (1006). In addition or in the alternative, deflation button (1006) may enable the operator to manually control the deflation of dilator (22). For instance, deflation button (1006) may activate pump (206) to draw fluid from dilation catheter (204) to thereby inflate dilator (22) for however long the operator holds deflation button (1006) in a depressed state. In some such versions, once the operator releases deflation button (1006), inflator assembly (202) may stop deflating dilator (22) and maintain the fluid pressure in dilation catheter (204) until the operator provides further input. It should also be understood that deflation button (1006) may provide different responses based on how the operator activates inflation button (204). For instance, deflation button (1006) may cause control module (208) automatically execute a selected fluid pressure profile in response to the operator quickly tapping deflation button (1006) one or two times; and provide manual deflation of dilator (22) in response to the operator holding deflation button (1006) in a depressed state. Other suitable ways in which deflation button (1006) may operate will be apparent to those of ordinary skill in the art in view of the teachings herein. In some versions where deflation of dilator (22) is fully automated as described further below, deflation button (1006) is omitted.

Rapid deflation button (1008) is operable to activate a high speed deflation mode as described above. In particular, rapid deflation button (1008) is operable to activate pump (206) to rapidly draw fluid from dilation catheter (204) to thereby deflate dilator (22). The deflation rate provided by rapid deflation button (1008) may be significantly greater than the deflation rate provided by deflation button (1006).

Back button (1010) is operable to change the display screen of indicators and/or inputs back to a previously displayed screen of indicators and/or inputs. Various examples of other indicators and inputs that may be included on a previously displayed screen will be described in greater detail below, while still other examples will be apparent to those of ordinary skill in the art in view of the teachings herein.

Warning indicator (1020) is configured to alert the user to one or more error conditions. By way of example only, warning indicator (1020) may illuminate to indicate a fault condition somewhere within system (200). In some versions, warning indicator (1020) may flash in various patterns to indicate specific fault conditions or other problems.

Inflation status indicator (1022) is configured to indicate whether dilator (22) is currently inflating or deflating. In the present example, inflation status indicator (1022) includes a textual message that states "inflating" or "deflating," in combination with an up arrow (which illuminates during inflation of dilator (22)) and a down arrow (which illuminates during deflation of dilator (22)).

Pressure indicator (1024) is configured to indicate the current pressure of fluid in dilator (22) in real time. This real time fluid pressure data may be acquired through a pressure sensor, force sensor, and/or other sensor(s) as described elsewhere herein.

Process completion indicator (1026) is configured to indicate when a dilation process is complete. By way of example only, process completion indicator (1026) may illuminate when system (200) has completed one or more cycles of inflating/deflating dilator (22), in accordance with any of the various operations described in greater detail below or in accordance with any other suitable operations. It should also be understood that inflator assembly (202) may provide an audible tone and/or some other form of feedback to complement activation of process completion indicator (1026), to further indicate to the operator that the dilation process is complete.

Unhook balloon indicator (1028) is configured to indicate to the operator that dilation catheter (204) may be detached from inflator assembly (202). By way of example only, unhook balloon indicator (1028) may illuminate when the fluid pressure in dilator (22) falls below a certain threshold after the dilation process is complete. It should be understood that, in some versions, unhook balloon indicator (1028) may be activated shortly after process completion indicator (1026) is activated. In versions where process completion indicator (1026) and unhook balloon indicator (1028) are both supplemented by audible tones, the audible tones may differ to further enable the operator to differentiate between the stage when the dilation process is complete and the subsequent stage when dilation catheter (204) may be detached from inflator assembly (202).

Treatment in progress indicator (1030) is configured to indicate to the operator that the dilation process is underway and ongoing. By way of example only, treatment in progress indicator (1030) may illuminate as soon as the operator activates inflation button (1004); may stay illuminated throughout the various stages of the dilation process; and may de-illuminate either upon activation of the process completion indicator (1026) or the unhook balloon indicator (1028).

Maximum pressure indicator (1032) is configured to show the maximum fluid pressure that is to be reached in dilator (22) in accordance with the selected fluid pressure profile. It should be understood that the operator may view pressure indicator (1024) in conjunction with maximum pressure indicator (1032) to monitor how close the actual fluid pressure is to the maximum fluid pressure.

Inflation duration indicator (1034) is configured to show how long dilator (22) is at an inflated state. In some versions, inflation duration indicator (1034) starts tracking time as soon as dilator (22) begins to inflate. In some other versions, inflation duration indicator (1034) starts tracking time as soon as the fluid pressure in dilator (22) reaches the maximum pressure amount in accordance with the selected fluid pressure profile. In either such versions, it should be understood that inflation duration indicator (1034) may reset to zero as soon as dilator (22) starts deflating from an inflated state. Alternatively, inflation duration indicator (1034) may reset to zero as soon as dilator (22) reaches a deflated state.

In addition to or in lieu of the input features described above, GUI (1000) may also include one or more other user input features. Such other user input features may enable the operator to select from various fluid pressure profiles. This may include features enabling the operator to provide various inputs (e.g., clinical context, measured ostia size, particular passageway to be dilated, etc.) that are processed in order to automatically select the most appropriate fluid pressure profiles from the various stored fluid pressure profiles. Additional user input features may also enable the operator to select the size and/or type of dilator (22) (e.g., by toggling through a predefined list, etc.). Additional input features may also enable the operator to select particular fluid pressure values (e.g., the maximum fluid pressure that will be displayed in maximum pressure indicator (1032), etc.). Additional input features may also enable the operator to select a particular inflation duration (e.g., the duration through which dilator (22) will be kept at the maximum fluid pressure, etc.). Other suitable input features that may be provided through GUI (1000) will be apparent to those of ordinary skill in the art in view of the teachings herein.

Similarly, in addition to or in lieu of the user feedback features described above, GUI (1000) may also include one or more other user feedback features or indicators. This may include features indicating the size of dilator (22) selected, the total time elapsed during the dilation process, and/or other information. Other suitable user feedback features or indicators that may be provided through GUI (1000) will be apparent to those of ordinary skill in the art in view of the teachings herein.

While control module (208) and user input feature (210) are both provided through laptop computer (264) in this example, control module (208) and user input feature (210) may alternatively be provided through any other suitable kinds of devices. By way of example only, control module (208) and user input feature (210) may alternatively be provided through a tablet device. In some other versions, user input feature (210) and/or control module (208) are provided through a dedicated console that is custom made for dilator system (200). It should also be understood that control module (208) and user input feature (210) may be integrated into the same device (e.g., as they are in computer (264)); or be provided by separate devices. Various suitable kinds of devices that may be used to provide user input feature (210) and control module (208) will be apparent to those of ordinary skill in the art in view of the teachings herein.

In the present example, dilation catheter (204) is in fluid communication with the pump (206) via tubing (278). More particularly, tubing extends between port (220) of syringe barrel (214) and lateral port (26) of dilation catheter (204). Shaft of dilation catheter (204) defines an internal lumen (32) leading to dilator (22). Thus, pump (206) is in fluid communication with lumen (32) and dilator (22) via tubing (278), and dilator (22) may receive fluid from reservoir (218) as plunger (216) is advanced into barrel (214).

In an exemplary use of dilator system (200), an operator may start with plunger (216) advanced to a distal position in barrel (214). The operator may then position port (220) in a bowl or other container of fluid, such as saline (not shown), from which to draw fluid. In instances where port (220) is coupled with one end of tubing (278), the operator may position the other end of tubing (278) in the saline. In either case, the operator may then retract plunger (216) relative to barrel (214) to draw the saline (or other fluid) into reservoir (218). The operator or clinician may fill reservoir (218) with saline (or other fluid) prior to or after mounting the syringe barrel (214) and handle feature (232) relative to the control module (208). For example, the operator may fill the reservoir (218) as just described, and subsequently couple the syringe assembly (212) to the first and second engagement features (238, 240) as described herein.

Before or after mounting syringe assembly (212) to drive mechanism (235), the operator may advance plunger (216) distally in order to purge air from reservoir (218). For instance, the operator may orient inflator (150) such that port (220) is positioned upwardly to gather air at the top of reservoir (218) before advancing plunger (216) distally in order to purge air from reservoir (218). The purging process may also be automated such that portions of system (200) such as control module (208) and pump (206) operate to fill the reservoir (218) with saline. In some such examples, filling the barrel (218) with saline may commence upon a button being pressed on the user input feature (210); and may cease upon one or more conditions in the system (200) being met. For instance, second engagement feature (240) may advance plunger (216) a predetermined distance in barrel (218) to purge air from barrel (218). Regardless of whether the air purge is initiated and/or driven manually or automatically, the system (200) may include a sensor that is configured to detect completion of air purging. Such a sensor may communicate to the control module (208) to cease operation of pump (206) once purging has completed.

Once reservoir (218) has been sufficiently filled with fluid and air has been purged, the operator may couple pump (206) with dilation catheter (204), such as by coupling port (220) with lateral port (26) via a flexible tube (278). The operator or clinician may then advance the dilation catheter (204) into a patient's head according to the teachings described herein, in order to position dilator (22) within an anatomical passageway (e.g., an ostium (O), etc.).

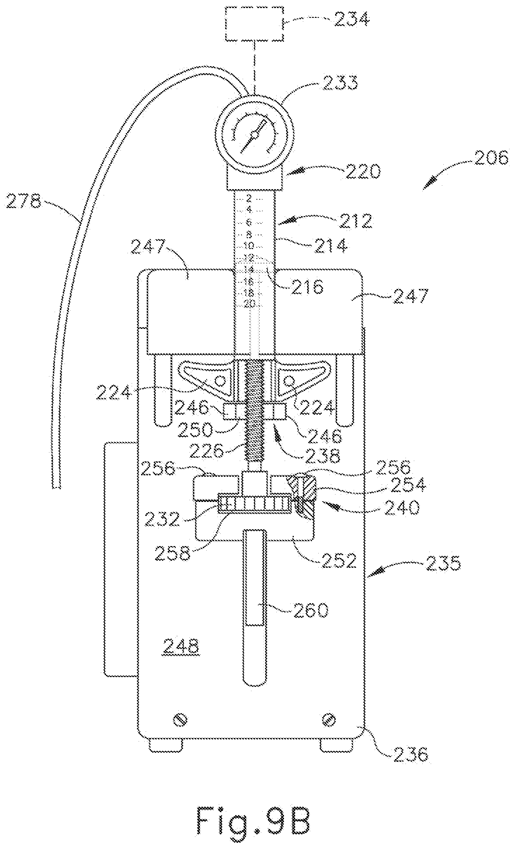

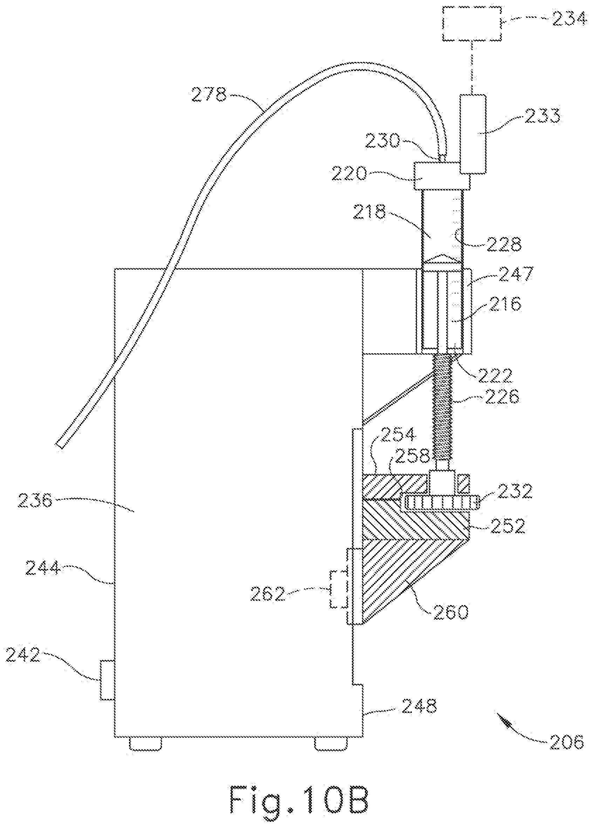

With dilator (22) being suitably positioned within an anatomical passageway (e.g., an ostium (O), etc.), the operator may then utilize the user input feature (210) to activate the control module (208) according to a particular fluid pressure profile. In that regard, the operator presses inflation button (204) to activate the actuator (262), causing actuator (262) to advance plunger (216) in barrel (214), such that plunger (216) moves from a first position (FIGS. 9A, 10A) toward a second position (FIGS. 9B, 10B). As plunger (216) advances in barrel (214), fluid transfers from reservoir (218) to dilator (22), thereby expanding dilator (22) in the anatomical passageway. Plunger (216) is advanced by the actuator (262) until reaching the second position where the hydraulic circuit reaches one or more conditions associated with the fluid pressure profile. As described below, system (200) may include various kinds of features that are operable to provide feedback to control module (208) that is indicative of the fluid pressure in the hydraulic circuit that includes dilator (22).

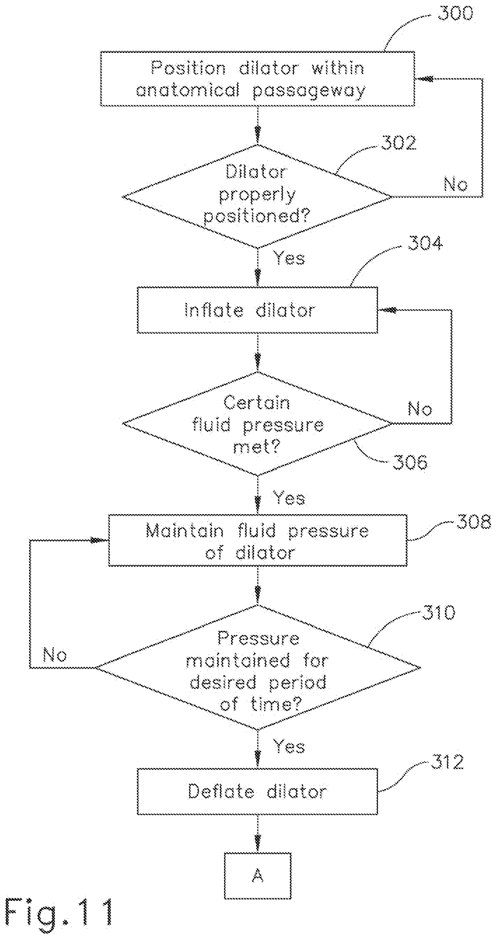

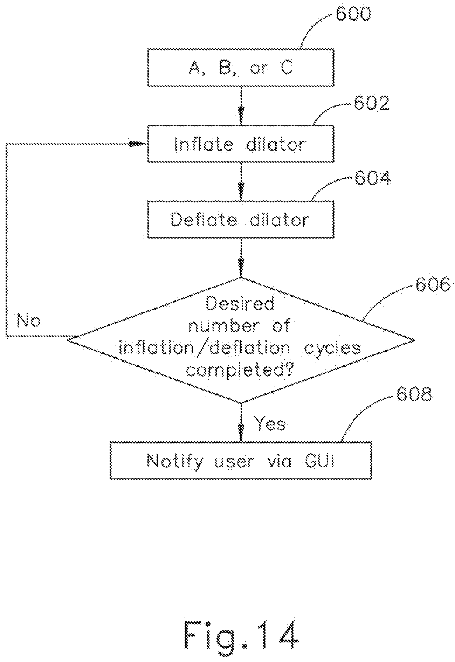

In the present example, and as shown in FIG. 11, the operator positions dilator (22) within the anatomical passageway (e.g., an ostium (O), etc.) (block 300) and ensures that the dilator (22) is properly positioned (block 302). With dilator (22) being suitably positioned within an anatomical passageway, the operator may then utilize the user input feature (210) to activate the control module (208) according to a particular fluid pressure profile, in order to inflate the dilator (22) (block 304). In that regard, the operator presses inflation button (204) to activate the actuator (262), causing actuator (262) to advance plunger (216) in barrel (214), such that plunger (216) moves from the first position (FIGS. 9A, 10A) toward the second position (FIGS. 9B, 10B). As plunger (216) advances in barrel (214), fluid transfers from reservoir (218) to dilator (22), thereby expanding dilator (22) in the anatomical passageway. The pressure sensor (234) monitors the fluid pressure level in the hydraulic circuit and provides real time feedback to control module (208). Control module (208) tracks the fluid pressure level in the hydraulic circuit (block 306) and commands drive mechanism (235) of pump (206) to cease movement and maintain the position of actuator (262) once the fluid pressure reaches a predetermined value in accordance with the selected pressure profile (block 306). When the position of actuator (262) is maintained, plunger (216) remains in the second position; and the predetermined fluid pressure level associated with the chosen fluid pressure profile is maintained within the dilation catheter (204) (block 308). Thus, dilator (22) remains inflated within the anatomical passageway at the desired fluid pressure. As described in greater detail below, this fluid pressure may be maintained for a selected or desired duration (block 310); then relieved to deflate dilator (22) (block 312). The process may be repeated any desired number of times. Merely illustrative examples of methods for repeating this process are shown in FIGS. 14-16 and are described in greater detail below.

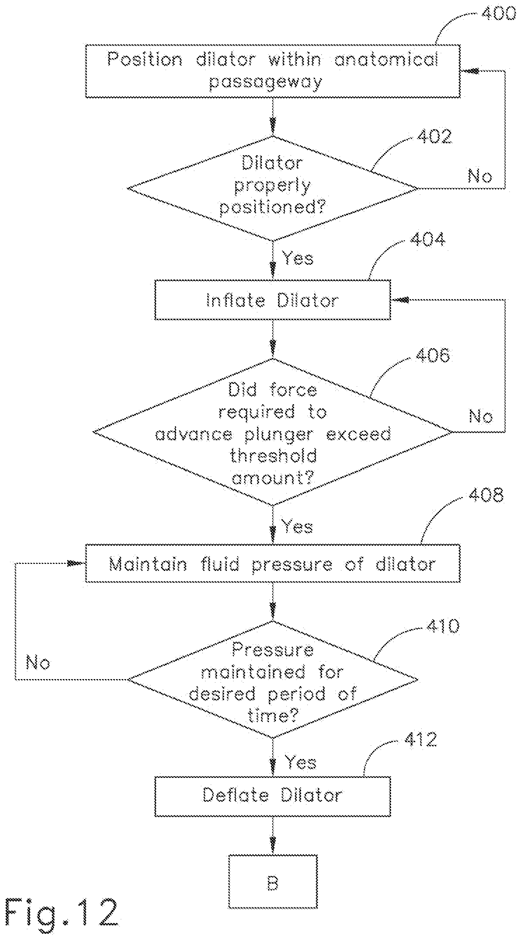

In the variation shown in FIG. 12, the operator positions dilator (22) within the anatomical passageway (e.g., an ostium (O), etc.) (block 400) and ensures that the dilator is properly positioned (block 402). With dilator (22) being suitably positioned within an anatomical passageway, the operator may then utilize the user input feature (210) to activate the control module (208) according to a particular fluid pressure profile, in order to inflate the dilator (22) (block 404). In that regard, the operator presses inflation button (204) to activate the actuator (262), causing actuator (262) to advance plunger (216) in barrel (214), such that plunger (216) moves from the first position (FIGS. 9A, 10A) toward the second position (FIGS. 9B, 10B). As plunger (216) advances in barrel (214), fluid transfers from reservoir (218) to dilator (22), thereby expanding dilator (22) in the anatomical passageway. In the variation shown in FIG. 12, the second position of plunger (216) is associated with a resistance force borne by plunger (216) against actuator (262). In some such versions, system (200) includes a force sensor in communication with plunger (216), barrel (214), and/or actuator (262), in addition to or in lieu of including a fluid pressure sensor (234). Such a force sensor may provide real time feedback to control module (208) as plunger (216) is advanced in barrel (214). Various suitable forms that such a force sensor may take will be apparent to those of ordinary skill in the art in view of the teachings herein.

Continuing with the example shown in FIG. 12, it should be understood that the resistance force borne by plunger (216) against actuator (262) during advancement of plunger (216) in barrel (214) may increase as the fluid pressure in the hydraulic circuit increases. Thus, the level of force required to advance plunger (216) in barrel (214) may be indicative of the pressure of fluid in the hydraulic circuit. Once the force sensor and control module (208) detect that the force required to advance plunger (216) in barrel (214) has reached a predetermined value associated with a fluid pressure value in accordance with the selected pressure profile (block 406), control module (208) commands the actuator (262) to cease movement and maintain its present position (block 408). As noted above, dilator (22) remains inflated within the anatomical passageway at the desired fluid pressure. As described in greater detail below, this fluid pressure may be maintained for a selected or desired duration (block 410); then relieved to deflate dilator (22) (block 412). Control module (208) may include a timer to track the duration. The process may be repeated any desired number of times. Merely illustrative examples of methods for repeating this process are shown in FIGS. 14-16 and are described in greater detail below.

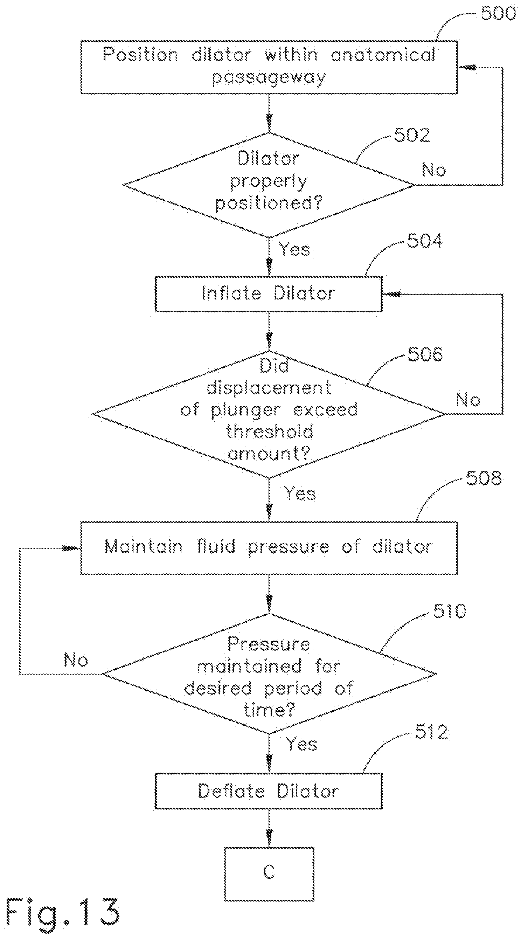

In the variation shown in FIG. 13, the operator positions dilator (22) within the anatomical passageway (e.g., an ostium (O), etc.) (block 500) and ensures that the dilator (22) is properly positioned (block 502). With dilator (22) being suitably positioned within an anatomical passageway, the operator may then utilize the user input feature (210) to activate the control module (208) according to a particular fluid pressure profile, in order to inflate the dilator (22) (block 504). In that regard, the operator presses inflation button (204) to activate the actuator (262), causing actuator (262) to advance plunger (216) in barrel (214), such that plunger (216) moves from the first position (FIGS. 9A, 10A) toward the second position (FIGS. 9B, 10B). As plunger (216) advances in barrel (214), fluid transfers from reservoir (218) to dilator (22), thereby expanding dilator (22) in the anatomical passageway. In the variation shown in FIG. 13, the second position of plunger (216) is associated with a predetermined displacement distance of actuator (262) and/or plunger (216) relative to barrel (214). Thus, control module (208) may simply track the displacement distance of actuator (262) and/or plunger (216) relative to barrel (214) to determine when the hydraulic circuit has achieved a predetermined fluid pressure in accordance with the selected pressure profile. Once the force sensor and/or control module (208) detect that the distance of displacement of the plunger (216) has reached a predetermined value associated with a fluid pressure value in accordance with the selected pressure profile (block 506), control module (208) commands the actuator (262) to cease movement and maintain its present position (block 508). As noted above, dilator (22) remains inflated within the anatomical passageway at the desired fluid pressure. As described in greater detail below, this fluid pressure may be maintained for a selected or desired duration (block 510); then relieved to deflate dilator (22) (block 512). The process may be repeated any desired number of times. Merely illustrative examples of methods for repeating this process are shown in FIGS. 14-16 and are described in greater detail below.

Various suitable components that may be used to track displacement of actuator (262) and/or plunger (216) relative to barrel (214) will be apparent to those of ordinary skill in the art in view of the teachings herein. It will be understood that the first and second positions of the plunger (216) as described herein are terms of relativity; and that, in practice, these positions may vary significantly from those shown in FIGS. 9A, 10A and FIGS. 9B and 10B, respectively.

Once the operator has attained the desired level of pressure in dilator (22) within the anatomical passageway to dilate the anatomical passageway, the operator may pause for an approximate, predetermined period of time (e.g., approximately three seconds, etc.). Operator may then depress inflation button (204) on the user input feature (210), which commands the actuator (262) to retract plunger (216) relative to barrel (214), to return the plunger (216) to the first position or to a position between the first position and the second position, to thereby relieve pressure in the hydraulic circuit and thus fully or partially deflate dilator (22). It should also be understood that the above described process of expanding and deflating dilator (22) may be repeated any desired number of times while dilator (22) is positioned in the same anatomical passageway. Alternatively, if the operator wishes to dilate additional anatomical passageways, dilator (22) may be positioned in the next anatomical passageway, and the operator may repeat the above steps to dilate that next anatomical passageway. Thus, the same volume and pressure of fluid within reservoir (218) may be used repeatedly, if desired, to dilate a plurality of anatomical passageways, without having to withdraw dilator (22) from the patient, and without having to decouple inflator assembly (202) from dilation catheter (204), until all of the desired dilations have been completed. In some versions, the desired number of dilations that are performed may be predetermined, according to a certain number; or, alternatively, according to different conditions that the system (10) detects or achieves.

In some versions, dilator system (200) is more fully automated and maintains the fluid pressure in an inflated dilator (22) for a predetermined period of time (e.g., three seconds, ten seconds, etc.), based on the selected pressure profile. Once the predetermined period of time has elapsed, control module (208) may automatically retract plunger (216) relative to barrel (214) to relieve the pressure in the hydraulic circuit, thereby deflating dilator (22). Dilator system (200) may also automatically repeat the expansion and deflation of dilator (22), automatically advancing and retracting plunger (216) (between the first and second positions) repeatedly until a desired amount of dilation in a particular anatomical passageway is achieved, based on the selected pressure profile. For example, referring to FIG. 14, once dilator (22) has been inflated and deflated through a first cycle, such as in any of the manners described above with respect to FIGS. 11-13 (block 600), dilator (22) may be re-inflated (block 602) and subsequently deflated (block 604) until a desired number of inflation and deflation cycles have been completed (block 606). The completion of a desired number of inflation and deflation cycles may be sensed automatically (e.g., by control module (208), etc.) in any manner apparent to those of ordinary skill in the art in view of the teachings herein. In the present example, control module (208) may stop the cycles after actuator (262) has been actuated a certain predetermined number of times (block 606). Alternatively, the operator may manually count the number of cycles completed. Upon the system (10) reaching the predetermined number of inflation/deflation cycles, the operator may be notified via the graphical user interface (block 608).

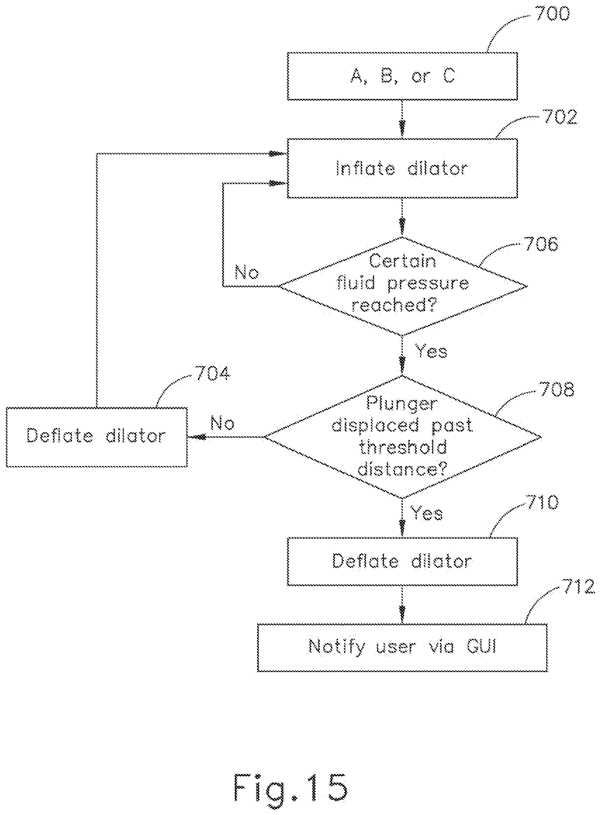

In some other versions, as shown in FIG. 15, once dilator (22) has been inflated and deflated through a first cycle, such as in any of the manners described above with respect to FIGS. 11-13 (block 700), dilator (22) may be re-inflated (block 702) and subsequently deflated (block 704) until certain characteristics or conditions are met by system (10). In the variation shown in FIG. 15, control module (208) could monitor the relationship between sensed fluid pressure (e.g., certain fluid pressure being reached in system (10)) (block 706) and distance traveled by actuator (262). For instance, once actuator (262) has to travel beyond a predetermined threshold displacement distance (block 708) to achieve a certain fluid pressure in the hydraulic circuit, this may indicate that the anatomical passageway is no longer providing substantial resistance to inflation of dilator (22), such that the anatomical passageway has been sufficiently dilated. Upon detecting this predetermined relationship between travel distance of actuator (262) (block 708) and fluid pressure (block 706), control module (208) may automatically stop the cyclical advancement and retraction of the actuator (262). However, until the actuator (262) travels beyond the predetermined threshold distance to achieve the certain fluid pressure in the hydraulic circuit, the control module (208) may continue the cyclical advancement and retraction of the actuator (262). Upon the system (10) reaching the predetermined threshold distance condition, the dilator may be deflated (block 710) and the operator may be notified via the graphical user interface (block 712).

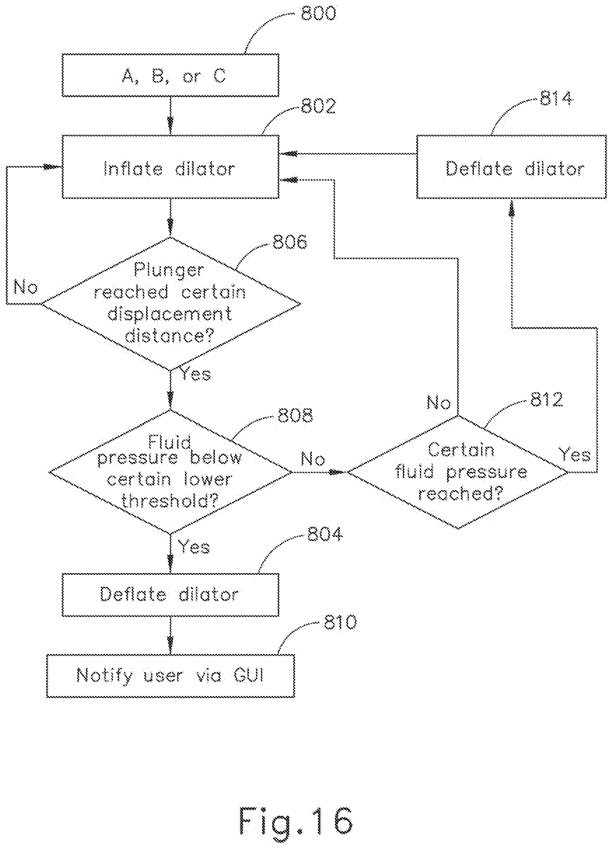

As yet another merely illustrative variation, as shown in FIG. 16, once dilator (22) has been inflated and deflated through a first cycle, such as in any of the manners described above with respect to FIGS. 11-13 (block 800), dilator (22) may be re-inflated (block 802) and subsequently deflated (block 804) until certain other characteristics or conditions are met by system (10). In the variation shown in FIG. 16, control module (208) could monitor the relationship between sensed fluid pressure (e.g., certain fluid pressure being reached in system (10)) and distance traveled by actuator (262). For instance, once actuator (262) has reached a certain predetermined threshold displacement distance (block 806), the control module (208) may observe whether the fluid pressure in the hydraulic circuit is below a threshold level (block 808). If the fluid pressure is below a certain threshold, dilator (22) may be deflated (block 804) and the operator may be notified via the graphical user interface (block 810). This is because the pressure being below predetermined threshold amount after the actuator (262) has reached a certain displacement distance (block 806) may indicate that the anatomical passageway is no longer providing substantial resistance to inflation of dilator (22), such that the anatomical passageway has been sufficiently dilated. However, if the fluid pressure is not below a certain predetermined threshold amount (block 808), the control module (208) may determine whether a certain fluid pressure has been reached (block 812). If the certain fluid pressure has not yet been reached, the control module (208) will continue advancing the actuator (262) in order to inflate dilator (22) (block 802). However, if the certain fluid pressure has been reached (block 812), the dilator (22) may be deflated (block 814), and another cyclical inflation and deflation cycle may be commenced, beginning at block 802.

In versions where dilator system (200) maintains the fluid pressure in an inflated dilator (22) for a predetermined period of time, then automatically relieves the pressure to deflate dilator (22), dilator system (200) may provide the operator with real time status information through user input feature (210). For instance, dilator system (200) may display the fluid pressure; the duration for which dilator (22) is inflated; the changes in state from inflating, to inflated, to deflating, to deflated; and/or any other kind of status information. In versions where dilator system (200) automatically repeats the inflation/deflation process and stops the process when control module (208) determines that the process is complete, dilator system (200) may also notify the operator via user input feature (210) that the dilation sequence is complete. This may prompt the operator to remove dilator (22) from the patient. Other suitable ways in which dilator system (200) may provide audio and/or visual feedback to the operator will be apparent to those of ordinary skill in the art in view of the teachings herein.

### IV. Miscellaneous

While examples herein are provided mainly in the context of dilating paranasal sinus ostia, it should be understood that dilator system (200) may also be used to dilate various other passageways. By way of example only, dilator system (200) may be used to dilate other fluid drainage passageways within the nasal cavity; dilation of the larynx, trachea, or other regions in a patient's airway; dilation of the Eustachian tube; and/or dilation of other passageways within the ear, nose, or throat, etc.

It should be understood that any of the examples described herein may include various other features in addition to or in lieu of those described above. By way of example only, any of the examples described herein may also include one or more of the various features disclosed in any of the various references that are cited herein.

It should be understood that any one or more of the teachings, expressions, embodiments, examples, etc. described herein may be combined with any one or more of the other teachings, expressions, embodiments, examples, etc. that are described herein. The above-described teachings, expressions, embodiments, examples, etc. should therefore not be viewed in isolation relative to each other. Various suitable ways in which the teachings herein may be combined will be readily apparent to those of ordinary skill in the art in view of the teachings herein. Such modifications and variations are intended to be included within the scope of the claims.

Versions of the devices disclosed herein can be designed to be disposed of after a single use, or they can be designed to be used multiple times. Versions may, in either or both cases, be reconditioned for reuse after at least one use. Reconditioning may include any combination of the steps of disassembly of the device, followed by cleaning or replacement of particular pieces, and subsequent reassembly. In particular, versions of the device may be disassembled, and any number of the particular pieces or parts of the device may be selectively replaced or removed in any combination. Upon cleaning and/or replacement of particular parts, versions of the device may be reassembled for subsequent use either at a reconditioning facility, or by a surgical team immediately prior to a surgical procedure. Those skilled in the art will appreciate that reconditioning of a device may utilize a variety of techniques for disassembly, cleaning/replacement, and reassembly. Use of such techniques, and the resulting reconditioned device, are all within the scope of the present application.

By way of example only, versions described herein may be processed before surgery. First, a new or used instrument may be obtained and if necessary cleaned. The instrument may then be sterilized. In one sterilization technique, the instrument is placed in a closed and sealed container, such as a plastic or TYVEK bag. The container and instrument may then be placed in a field of radiation that can penetrate the container, such as gamma radiation, x-rays, or high-energy electrons. The radiation may kill bacteria on the instrument and in the container. The sterilized instrument may then be stored in the sterile container. The sealed container may keep the instrument sterile until it is opened in a surgical facility. A device may also be sterilized using any other technique known in the art, including but not limited to beta or gamma radiation, ethylene oxide, or steam.

Having shown and described various versions of the present invention, further adaptations of the methods and systems described herein may be accomplished by appropriate modifications by one of ordinary skill in the art without departing from the scope of the present invention. Several of such potential modifications have been mentioned, and others will be apparent to those skilled in the art. For instance, the examples, versions, geometrics, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.

## Claims

1. An inflator system (100, 200), comprising:
(a) a dilation catheter (104, 204), wherein the dilation catheter comprises:
(i) a shaft defining a lumen, wherein the shaft is sized to fit within an anatomical passageway in a patient, and
(ii) a dilator (22) in fluid communication with the lumen, wherein the dilator (22) is configured to receive fluid through the lumen and thereby expand in response to fluid communicated through the lumen;
(b) a fluid pressure regulator assembly (102, 202), wherein the fluid pressure regulator assembly comprises:
(i) a pump (106, 206) in fluid communication with the lumen of the shaft,
(ii) a control module (108, 208), wherein the control module (108, 208) is configured to store one or more fluid pressure profiles, wherein the control module (108, 208) is operable to receive a selection of a particular fluid pressure profile from the one or more of fluid pressure profiles, wherein the control module (108, 208) is operable to selectively drive the pump (106, 206) via an actuator (262) in accordance with a selected fluid pressure profile to inflate the dilator (22), and
(iii) a user input feature (110, 210) operable to selectively activate the control module (108, 208); and
(c) a pressure sensor (112, 234) configured to sense a pressure of fluid in the dilation catheter (104, 204) or another portion of the inflator system, wherein the pressure sensor (112, 234) is in communication with the control module (108, 208); and
wherein the control module (108, 208) is configured to drive the pump (106, 206) to automatically repeat inflation and deflation of the dilator (22) until a condition based on the relationship between sensed fluid pressure and distance travelled by the actuator (262) is met.

2. The inflator system of claim 1, wherein the at least one of the one or more fluid pressure profiles is associated with attaining a predetermined fluid pressure within the dilation catheter.

3. The inflator system of claim 1, wherein at least one of the one or more fluid pressure profiles is associated with expanding the dilator to a predetermined cross-sectional dimension.

4. The inflator system of claim 1, wherein at least one of the one or more fluid pressure profiles is associated with displacing a component of the pump (106, 206) a predetermined distance.

5. The inflator system of claim 1, wherein the pump (106, 206) further comprises:
(A) a syringe barrel (214) in fluid communication with the shaft; and
(B) a syringe plunger (216) configured to move within the barrel to increase or decrease an amount of fluid delivered to the dilator, wherein the plunger (216) is operably coupled to the control module.

6. The inflator system of claim 5, wherein the syringe plunger (216) further comprises a syringe plunger handle (232), wherein the pump (106, 206) further comprises an engagement feature (240) to engage at least a portion of the syringe plunger handle (232).

7. The inflator system of claim 6, wherein the engagement feature (240) is configured to at least partially envelop the syringe plunger handle (232).

8. The inflator system of claim 5, wherein the pump (106, 206) further comprises an engagement feature (240) configured to maintain a position of the barrel (214) as the plunger (214) is moved within the barrel (214).

9. The inflator system of claim 5, wherein the actuator is operably coupled to the plunger (216) and configured to drive the plunger (216) in a first direction and in a second direction.

10. The inflator system of claim 9, wherein the actuator (262) comprises a linear actuator.

11. The inflator system of claim 1, wherein the plunger (216) is movable between a first position and a second position, wherein at least the second position is associated with at least one of the one or more fluid pressure profiles.

12. The inflator system of claim 1, further comprising a force sensor configured to sense a force required to drive the pump, wherein the force sensor is in communication with the control module.

13. The inflator system of claim 1, wherein the user input feature further comprises one of the following:
a computer device having a touch screen interface, or a computer device including a monitor in communication with a mouse and/or keyboard.

14. The inflator system of claim 1 wherein the condition comprises the actuator (262) travelling at least a predetermined threshold distance to achieve a certain fluid pressure.

15. The inflator system of claim 1 wherein the condition comprises the fluid pressure being below a predetermined threshold level when the actuator (262) has moved a predetermined distance.

## Patentansprüche

1. Aufblähsystem (100, 200), umfassend:
(a) einen Dilationskatheter (104, 204), wobei der Dilationskatheter umfasst:
(i) einen Schaft, der ein Lumen definiert, wobei der Schaft dafür dimensioniert ist, in einen anatomischen Durchgang in einem Patienten zu passen, und
(ii) einen Dilator (22) in Fluidverbindung mit dem Lumen, wobei der Dilator (22) dafür gestaltet ist, Fluid durch das Lumen aufzunehmen und sich in Antwort auf durch das Lumen zugeführtes Fluid auszudehnen;
(b) eine Fluiddruckregulatorbaugruppe (102, 202), wobei die Fluiddruckregulatorbaugruppe umfasst:
(i) eine Pumpe (106, 206) in Fluidverbindung mit dem Lumen des Schafts,
(ii) ein Steuermodul (108, 208), wobei das Steuermodul (108, 208) dafür gestaltet ist, ein oder mehrere Fluiddruckprofile zu speichern, wobei das Steuermodul (108, 208) funktionsfähig ist, ein ausgewähltes bestimmtes Fluiddruckprofil von dem einen oder den mehreren Fluiddruckprofilen zu erhalten, wobei das Steuermodul (108, 208) funktionsfähig ist, die Pumpe (106, 206) über einen Aktor (262) einem ausgewählten Fluiddruckprofil entsprechend selektiv zu steuern, um den Dilator (22) aufzublähen, und
(iii) ein Benutzereingabeelement (110, 210), das funktionsfähig ist, das Steuermodul (108, 208) selektiv zu aktivieren; und
(c) einen Drucksensor (112, 234), der dafür gestaltet ist, einen Fluiddruck in dem Dilationskatheter (104, 204) oder einem anderen Teil des Aufblähsystems zu erfassen,
wobei der Drucksensor (112, 234) mit dem Steuermodul (108, 208) verbunden ist; und
wobei das Steuermodul (108, 208) dafür gestaltet ist, die Pumpe (106, 206) zu treiben, um Aufblähen und Entleeren des Dilators (22) automatisch zu wiederholen, bis ein Zustand auf der Grundlage der Beziehung zwischen erfasstem Fluiddruck und von dem Aktor (262) zurückgelegtem Weg erfüllt ist.

2. Aufblähsystem gemäß Anspruch 1, wobei das wenigstens eine des einen oder der mehreren Fluiddruckprofile mit dem Erreichen eines vorbestimmten Fluiddrucks in dem Dilationskatheter verbunden ist.

3. Aufblähsystem gemäß Anspruch 1, wobei wenigstens eines des einen oder der mehreren Fluiddruckprofile mit dem Ausdehnen des Dilators auf eine vorbestimmte Querschnittsabmessung verbunden ist.

4. Aufblähsystem gemäß Anspruch 1, wobei wenigstens eines des einen oder der mehreren Fluiddruckprofile mit dem Verschieben einer Komponente der Pumpe (106, 206) um einen vorbestimmten Weg verbunden ist.

5. Aufblähsystem gemäß Anspruch 1, wobei die Pumpe (106, 206) ferner umfasst:
(A) einen Spritzenzylinder (214) in Fluidverbindung mit dem Schaft; und
(B) einen Spritzenkolben (216), der dafür gestaltet ist, sich in dem Zylinder zu bewegen, um die Menge an dem Dilator zugeführtem Fluid zu erhöhen oder zu verringern, wobei der Kolben (216) funktionsfähig an das Steuermodul gekoppelt ist.

6. Aufblähsystem gemäß Anspruch 5, wobei der Spritzenkolben (216) ferner ein Spritzenkolben-Griffstück (232) umfasst, wobei die Pumpe (106, 206) ferner ein Eingriffselement (240) zum Eingriff mit wenigstens einem Teil des Spritzenkolben-Griffstücks (232) umfasst.

7. Aufblähsystem gemäß Anspruch 6, wobei das Eingriffselement (240) dafür gestaltet ist, das Spritzenkolben-Griffstück (232) wenigstens teilweise zu umschließen.

8. Aufblähsystem gemäß Anspruch 5, wobei die Pumpe (106, 206) ferner ein Eingriffselement (240) umfasst, das dafür gestaltet ist, die Stellung des Zylinders (214) festzuhalten, wenn der Kolben (214) innerhalb des Zylinders (214) bewegt wird.

9. Aufblähsystem gemäß Anspruch 5, wobei der Aktor funktionsfähig an den Kolben (216) gekoppelt ist und dafür gestaltet ist, den Kolben (216) in eine erste Richtung und in eine zweite Richtung zu treiben.

10. Aufblähsystem gemäß Anspruch 9, wobei der Aktor (262) einen linearen Aktor umfasst.

11. Aufblähsystem gemäß Anspruch 1, wobei der Kolben (216) zwischen einer ersten Stellung und einer zweiten Stellung beweglich ist, wobei wenigstens die zweite Stellung mit wenigstens einem des einen oder der mehreren Fluiddruckprofile verbunden ist.

12. Aufblähsystem gemäß Anspruch 1, ferner umfassend einen Kraftsensor, der dafür gestaltet ist, eine zum Antreiben der Pumpe erforderliche Kraft zu erfassen, wobei der Kraftsensor mit dem Steuermodul verbunden ist.

13. Aufblähsystem gemäß Anspruch 1, wobei das Benutzereingabeelement ferner eines der folgenden umfasst:
eine Computervorrichtung mit einer Touchscreen-Schnittstelle oder eine Computervorrichtung, die eine Anzeige in Verbindung mit einer Maus und/oder einer Tastatur umfasst.

14. Aufblähsystem gemäß Anspruch 1, wobei der Zustand umfasst, dass sich der Aktor (262) um wenigstens eine vorbestimmte Grenzwertstrecke bewegt, um einen bestimmten Fluiddruck zu erzielen.

15. Aufblähsystem gemäß Anspruch 1, wobei der Zustand umfasst, dass der Fluiddruck unter einem vorbestimmten Grenzwert liegt, wenn sich der Aktor (262) um eine vorbestimmte Strecke bewegt hat.

## Revendications

1. Système de gonflage (100, 200), comprenant :
(a) un cathéter de dilatation (104, 204), le cathéter de dilatation comprenant :
(i) une tige définissant une lumière, la tige étant dimensionnée pour s'installer à l'intérieur d'un passage anatomique dans le corps d'un patient, et
(ii) un dilatateur (22) en communication fluidique avec la lumière, le dilatateur (22) étant configuré pour recevoir un fluide par la lumière et ainsi se dilater en réponse au fluide communiqué par la lumière ;
(b) un ensemble de régulation de pression de fluide (102, 202), l'ensemble de régulation de pression de fluide comprenant :
(i) une pompe (106, 206) en communication fluidique avec la lumière de la tige,
(ii) un module de commande (108, 208), le module de commande (108, 208) étant configuré pour stocker un ou plusieurs profils de pression de fluide, le module de commande (108, 208) étant utilisable pour recevoir une sélection d'un profil de pression de fluide particulier parmi le ou les profils de pression de fluide, le module de commande (108, 208) étant utilisable pour entraîner sélectivement la pompe (106, 206) par le biais d'un actionneur (262) en fonction d'un profil de pression de fluide sélectionné pour gonfler le dilatateur (22), et
(iii) un élément de saisie utilisateur (110, 210) utilisable pour activer sélectivement le module de commande (108, 208) ; et
(c) un capteur de pression (112, 234) configuré pour détecter une pression de fluide dans le cathéter de dilatation (104, 204) ou une autre partie du système de gonflage, le capteur de pression (112, 234) étant en communication avec le module de commande (108, 208) ; et
dans lequel le module de commande (108, 208) est configuré pour entraîner la pompe (106, 206) pour répéter automatiquement le gonflage et le dégonflage du dilatateur (22) jusqu'à ce qu'une condition basée sur la relation entre pression de fluide détectée et distance parcourue par l'actionneur (262) soit respectée.

2. Système de gonflage de la revendication 1, dans lequel l'au moins un du ou des profils de pression de fluide est associé à l'atteinte d'une pression de fluide prédéterminée à l'intérieur du cathéter de dilatation.

3. Système de gonflage de la revendication 1, dans lequel au moins un du ou des profils de pression de fluide est associé à la dilatation du dilatateur jusqu'à une dimension en coupe transversale prédéterminée.

4. Système de gonflage de la revendication 1, dans lequel au moins un du ou des profils de pression de fluide est associé au déplacement d'un composant de la pompe (106, 206) sur une distance prédéterminée.

5. Système de gonflage de la revendication 1, dans lequel la pompe (106, 206) comprend en outre :
(A) un cylindre de seringue (214) en communication fluidique avec la tige ; et
(B) un piston de seringue (216) configuré pour se déplacer à l'intérieur du cylindre pour augmenter ou diminuer une quantité de fluide délivrée au dilatateur, le piston (216) étant fonctionnellement couplé au module de commande.

6. Système de gonflage de la revendication 5, dans lequel le piston de seringue (216) comprend en outre une poignée de piston de seringue (232), la pompe (106, 206) comprenant en outre un élément de prise (240) pour entrer en prise avec au moins une partie de la poignée de piston de seringue (232).

7. Système de gonflage de la revendication 6, dans lequel l'élément de prise (240) est configuré pour envelopper au moins partiellement la poignée de piston de seringue (232).

8. Système de gonflage de la revendication 5, dans lequel la pompe (106, 206) comprend en outre un élément de prise (240) configuré pour maintenir une position du cylindre (214) lorsque le piston (214) est déplacé à l'intérieur du cylindre (214).

9. Système de gonflage de la revendication 5, dans lequel l'actionneur est fonctionnellement couplé au piston (216) et configuré pour entraîner le piston (216) dans une première direction et dans une deuxième direction.

10. Système de gonflage de la revendication 9, dans lequel l'actionneur (262) comprend un actionneur linéaire.

11. Système de gonflage de la revendication 1, dans lequel le piston (216) est mobile entre une première position et une deuxième position, au moins la deuxième position étant associée à au moins un du ou des profils de pression de fluide.

12. Système de gonflage de la revendication 1, comprenant en outre un capteur de force configuré pour détecter une force nécessaire pour entraîner la pompe, le capteur de force étant en communication avec le module de commande.

13. Système de gonflage de la revendication 1, dans lequel l'élément de saisie utilisateur comprend en outre un des éléments suivants :
un dispositif informatique ayant une interface à écran tactile, ou un dispositif informatique comportant un moniteur en communication avec une souris et/ou un clavier.

14. Système de gonflage de la revendication 1 dans lequel la condition comprend le fait que l'actionneur (262) parcourt au moins une distance seuil prédéterminée pour atteindre une certaine pression de fluide.

15. Système de gonflage de la revendication 1 dans lequel la condition comprend le fait que la pression de fluide est inférieure à un niveau seuil prédéterminé quand l'actionneur (262) s'est déplacé d'une distance prédéterminée.
